(19) Europäisches Patentamt
European Patent Office
Office européen des brevets

(11) Veröffentlichungsnummer: **0 050 781**
**B1**

(12) **EUROPÄISCHE PATENTSCHRIFT**

(45) Veröffentlichungstag der Patentschrift: **23.11.83**

(51) Int. Cl.[3]: **C 07 H 19/06**, C 07 H 19/04, A 01 N 43/50, A 01 N 43/54

(21) Anmeldenummer: **81108196.7**

(22) Anmeldetag: **12.10.81**

(54) **Nikkomycin-Salze, Verfahren zu ihrer Herstellung, diese enthaltende Schädlingsbekämpfungsmittel, ihre Herstellung und Verwendung.**

(30) Priorität: **25.10.80 DE 3040293**

(43) Veröffentlichungstag der Anmeldung: **05.05.82 Patentblatt 82/18**

(45) Bekanntmachung des Hinweises auf die Patenterteilung: **23.11.83 Patentblatt 83/47**

(84) Benannte Vertragsstaaten: **AT BE CH DE FR GB IT LI NL**

(56) Entgegenhaltungen:
**EP - A - 0 013 766**

**LIEBIGS ANN. CHEM. 1979, Seiten 1494-1502 H. HAGENMAIER et al.: *"Aufklärung der Struktur des Nucleosidantibiotikums Nikkomycin X"***

(73) Patentinhaber: **BAYER AG, Zentralbereich Patente, Marken und Lizenzen, D-5090 Leverkusen 1, Bayerwerk (DE)**

(72) Erfinder: **Moeschler, Heinrich, Ferdinand, Dr., Hahnenweg 8, D-5000 Köln 80 (DE)**
Erfinder: **Sasse, Klaus, Dr., Puetzweg 13, D-5060 Bergisch Gladbach 2 (DE)**
Erfinder: **Lange, Peter Michael, Dr., Walter-Flex-Strasse 9, D-5090 Leverkusen 1 (DE)**
Erfinder: **Gölker, Christan, Dr., Am Rohm 45, D-5600 Wuppertal 1 (DE)**
Erfinder: **Zoebelein, Gerhard, Dr., Domblick 46, D-5090 Leverkusen 31 (DE)**
Erfinder: **Telle, Otto, Haferkamp 10, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Nikkomycin-Salze, Verfahren zu ihrer Herstellung, diese enthaltende Schädlingsbekämpfungsmittel, ihre Herstellung und Verwendung

Die vorliegende Erfindung betrifft neue Cr-, Fe-, Co-, Ni-, Cu- und Zn-Salze von Nikkomycin (im folgenden teilweise kurz als Schwermetallsalze von Nikkomycin bezeichnet), ein neues Verfahren zu ihrer Herstellung, diese Salze enthaltende Schädlingsbekämpfungsmittel sowie ihre Verwendung als Schädlingsbekämpfungsmittel.

Nikkomycin, seine Herstellung auf mikrobiologischem Weg mittels des Stammes Streptomyces tendae Ettlingen (CBS 354.75) und seine Verwendung als Pflanzenschutzmittel sind bekannt (vgl. deutsche Offenlegungsschrift Nr. 25 370 28, US-Patentschriften Nr. 4 046 881 und 4 158 608). Es stellte sich heraus, dass das derart gewonnene Nikkomycin ein Stoffgemisch ist, das aus sich ähnelnden Stoffen besteht, welche Nikkomycine genannt werden. Die bei der Fermentation und Aufarbeitung gewonnenen Nikkomycin-Gemische entsprechen in ihren Hauptbestandteilen der Formel I

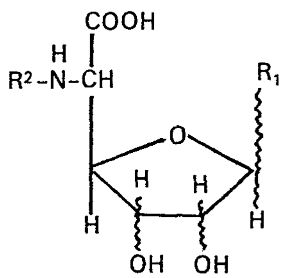

(I)

in der $R_1$ vor allem die Bedeutung

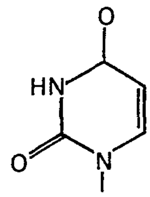

und

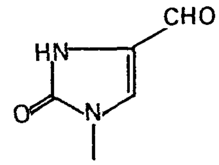

und $R_2$ vor allem die Bedeutung

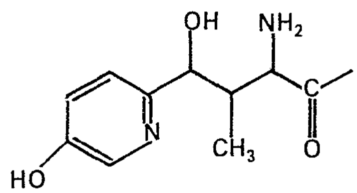

hat.

Nikkomycine sind wertvolle Mittel zur Bekämpfung von Pflanzenschädlingen und können beispielsweise als Insektizide, insbesondere Akarizide oder Fungizide verwendet werden. Hierbei haben sich die im Nikkomycin-Gemisch der mikrobiologischen Herstellung enthaltenen Nikkomycine (X) und (Z) sowie ihre Gemische als besonders bedeutsam erwiesen.

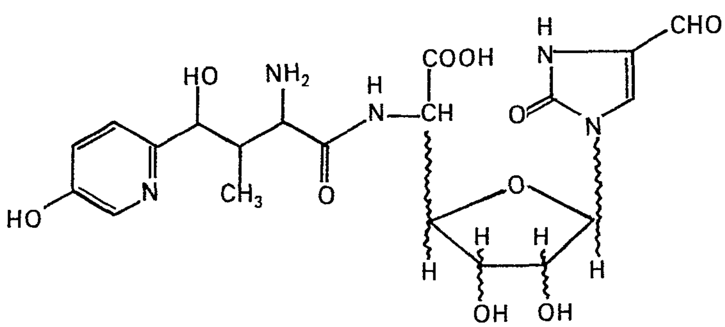

Nikkomycin (X)

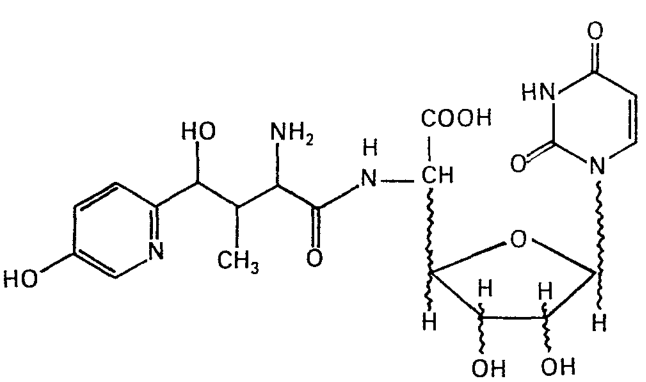

Nikkomycin (Z)

Wie bereits angedeutet, können Nikkomycine, insbesondere die Nikkomycine (X) und (Z) und ihre Mischungen zur Bekämpfung von Schädlingen, vorzugsweise als Fungizide sowie als Insektizide und insbesondere als Akarizide verwendet werden. Bei bestimmten Anwendungsformen

und unter ungünstigen feuchten Bedingungen, z.B. Witterungsverhältnissen, ist die Dauerwirkung der Nikkomycine jedoch nicht in allen Fällen voll befriedigend.

Die vorliegende Erfindung betrifft neue Cr-, Fe-, Co-, Ni-, Cu- und Zn-Salze von Nikkomycin, insbesondere der Nikkomycine (X) und (Z) und ihrer Mischungen (im folgenden teilweise kurz als Schwermetallsalze von Nikkomycin bezeichnet).

Besonders bevorzugte Nikkomycin-Schwermetallsalze sind die Zn- und Cu-Salze, insbesondere die Zn-Salze.

Die neuen Nikkomycin-Schwermetallsalze stellen pulvrige Stoffe dar, deren Lösung in wässriger 0,1n HCl gleiche Spektraleigenschaften aufweisen, wie eine Lösung von «freiem» Nikkomycin in 0,1n HCl, z.B. $UV_{\lambda max}$ (0,1n HCl) 230(sh)nm, 286nm.

Diese Methode kann zur Identifizierung der neuen Stoffe herangezogen werden.

Die neuen Nikkomycin-Schwermetallsalze werden dadurch erhalten, dass man Nikkomycin, vorzugsweise Nikkomycin (X) und (Z) und ihre Mischungen mit wasserlöslichen Cr-, Fe-, Co-, Ni-, Cu und Zn-Salzen in wässrigem Medium umsetzt.

Als Schwermetallsalze werden beim erfindungsgemässen Verfahren bevorzugt in Wasser lösliche Cu- und Zn-Salze, insbesondere Zn-Salze eingesetzt. Vorzugsweise werden in Wasser lösliche Schwermetallsalze verwendet, in welchen die Schwermetalle in 2- oder 3-wertiger Form vorliegen. Bevorzugt sind Schwermetall-Chloride, -Sulfate und -Acetate, insbesondere Sulfate.

Als Beispiele für erfindungsgemäss verwendbare Schwermetallsalze seien genannt:

$ZnCl_2$, $ZnSO_4 \times 7\,H_2O$, $Zn(CH_3COO)_2$,
$CuCl_2 \times 2\,H_2O$, $CuSO_4 \times 5\,H_2O$, $Cu(CH_3COO)_2$,
$Cu(NO_3)_2 \times 3\,H_2O$, $Cr_2(SO_4)_3 \times 18\,H_2O$,
$Fe(SO_4)$, $Fe_2(SO_4)_3$, $FeCl_3$, $FeCl_2$, $CoCl_2$,
$CoSO_4 \times 7\,H_2O$, $NiSO_4 \times 7\,H_2O$, $NiCl_2 \times 6\,H_2O$.

Art und Beschaffenheit des beim erfindungsgemässen Verfahrens einzusetzenden Nikkomycins sind weitestgehend unkritisch.

Das Nikkomycin kann in reiner Form oder in Form eines Rohnikkomycins vorliegen, welches bereits nach bekannten und/oder weiter unten beschriebenen Verfahren vorgereinigt wurde. Es kann auch die nicht vorgereinigte Rohnikkomycin-Lösung, wie sie als Kulturbrühe bei der mikrobiellen Nikkomycin-Erzeugung anfällt, gegebenenfalls nach ihrer Konzentrierung eingesetzt werden.

Die beim erfindungsgemässen Verfahren verwendeten Gewichtsverhältnisse der Ausgangsstoffe sind weitgehend unkritisch und können in Übereinstimmung mit der Art und Form der Ausgangsprodukte sowie den vorhandenen Geräten leicht bestimmt werden.

Im allgemeinen setzt man auf 1 Mol Nikkomycin 1 bis 10, vorzugsweise 1 bis 2 Mol, Schwermetallsalz ein. Die Molangabe zum Nikkomycin bezieht sich auf das in eingesetztem Rohprodukt enthaltene reine Nikkomycin (vor allem Nikkomycin [X] und [Z], wie es mit Hilfe der Hochdruck-Flüssig-Chromatographie (HPLC) nach bekannten Methoden ermittelt wurde.

Zweckmässigerweise wird zu einer wässrigen Lösung von Nikkomycin (vorzugsweise 1 kg Rohmikkomycin in etwa 2 bis 20, insbesondere 2 bis 4 Liter Wasser) das Schwermetallsalz gegebenenfalls in wässriger Lösung in Portionen zugegeben, wobei der pH-Wert während der Reaktion durch Basenzugabe auf Werte zwischen etwa 6,0 und etwa 7,5, vorzugsweise zwischen etwa 6,5 und etwa 7,0 gehalten wird. Als Base wird hierbei vorzugsweise eine anorganische wasserlösliche Base, wie NaOH oder KOH verwendet. Man gibt soviel Schwermetallsalz zu, bis kein weiterer Niederschlag gebildet wird. Auf diese Weise kann die erforderliche Menge Schwermetallsalz sehr einfach ermittelt werden.

Zweckmässigerweise gibt man zur Ausbeuteverbesserung das Schwermetallsalz in einer überschüssigen Menge zu und lässt das Reaktionsgemisch noch einige Stunden zur Nachreaktion stehen, bis sich kein zusätzlicher Niederschlag mehr bildet. Die Reaktion wird vorzugsweise bei Temperaturen zwischen 0 bis 50°C, insbesondere zwischen etwa 15 und 25°C vorgenommen.

Das ausgefällte Nikkomycin-Schwermetallsalz wird nach üblichen Methoden (Abfiltrieren, Abzentrifugieren) abgetrennt und gegebenenfalls mit Wasser und/oder einem organischen Lösungsmittel, z.B. Aceton gewaschen und anschliesend getrocknet.

Wie bereits oben dargelegt, kann das Nikkomycin als Ausgangsprodukt für das erfindungsgemässe Verfahren in gereinigter oder roher Form verwendet werden. So kann z.B. das gemäss der deutschen Offenlegungsschrift Nr. 2 537 028 und den US-Patentschriften Nr. 4 046 881 und 4 158 608 erhaltene Produkt eingesetzt werden.

Ein für das erfindungsgemässe Verfahren besonders vorteilhaft einsetzbares Nikkomycin mit hohem Nikkomycin (X)- und (Z)-Anteil kann z.B. erhalten werden, indem Rohnikkomycin oder eine Nikkomycin enthaltende Kulturbrühe mit einem basischen Ionenaustauscher behandelt wird, das (X)- und (Z)-Gemisch mit einer Säure eluiert und das erhaltene Produkt nach den üblichen Methoden isoliert wird.

Bevorzugt wird hierbei in einem ersten Schritt das nach der Fermentation erhaltene Kulturfiltrat mit einem sauren Ionenaustauscher behandelt. Bei anschliessender Elution mit Ammoniak wird eine Rohnikkomycin-Lösung gewonnen, welche für eine Anreicherung der Nikkomycine (X) und (Z) besonders gut geeignet ist.

Eine geeignete Rohnikkomycin-Lösung kann auch auf besonders günstige Weise, wie folgt, erhalten werden:

Falls das Rohnikkomycin nicht bereits als Lösung vorliegt oder ein Kulturfiltrat eingesetzt wird, wird das Rohnikkomycin in vorzugsweise entmineralisiertem oder destilliertem Wasser gelöst. Die Konzentration der Lösung ist unkritisch

und wird lediglich durch die Löslichkeit des Nikkomycins einerseits und dem Wunsch nach einer leicht handhabbaren Flüssigkeitsmenge (Grösse der Apparaturen) andererseits begrenzt. Die Lösung des Rohnikkomycins wird, falls nicht ein entsprechender pH-Wert bereits vorliegt, durch Säurezusatz auf einen pH-Wert von 4 bis 7, vorzugsweise von 6 bis 7 gebracht. Hierzu können alle üblichen anorganischen (z.B. HCl) und vorzugsweise organischen Säuren, insbesondere niedere aliphatische Carbonsäuren, wie Essigsäure oder Propionsäure verwendet werden, welche Nikkomycin nicht angreifen. Bevorzugt ist Essigsäure.

Die wässrige Lösung (welche auch organische Lösungsmittel, wie Methanol enthalten kann) wird in üblicher Weise mit dem basischen Ionenaustauscher in Kontakt gebracht (z.B. durch Aufgeben auf eine Säule oder durch Einrühren in einen Kessel). Die günstigste Menge an Ionenaustauscher ist abhängig von der eingesetzten Lösung und von der Art des Ionenaustauschers und kann nach üblichen Methoden leicht ermittelt werden.

Für diese Methode zur Herstellung des «Rohnikkomycins» kommen alle üblichen basischen Ionenaustauscher in Frage. Beispielhaft seien aufgeführt:

Basische makroporöse oder gelförmige, mit Divinylbenzol vernetzte Polystyrolharze, die teilweise oder vollständig mit primären, sekundären, tertiären oder quartären Stickstoffgruppen substituiert sind. Ebenso sind auch makroporöse und gelförmige Ionenaustauscher verwendbar, die sich von vernetztem Polyacrylamid ableiten.

Weiterhin können basische gelförmige oder makroporöse Ionenaustauscher auf Acryl- oder Methacrylsäureesterbasis, die mit z.B. Dimethylaminopropylamin umamidiert wurden, eingesetzt werden. Der funktionelle Stickstoff kann primär, sekundär, tertiär, quartär oder als Gemisch dieser Gruppierungen vorliegen. Besonders bevorzugt wird als schwach basischer Ionenaustauscher ein makroporöses aminomethyliertes Polystyrol, welches mit ca. 6% Divinylbenzol vernetzt ist (vgl. deutsche Patentschrift Nr. 24 18 976 und US-Patentschrift Nr. 3 989 650).

Als basische Ionenaustauscher können auch basische gelförmige Ionenaustauscher auf Dextranbasis eingesetzt werden.

Als spezielle Beispiele für basische Ionenaustauscher seien genannt: Lewatit MP 500 (Warenzeichen der BAYER AG, Leverkusen, Deutschland, BRD), Dowex MSA-1 (Warenzeichen der Dow Chemicals, USA) sowie DEAE-Sephadex A-25 und QAE-Sephadex A 25 (Warenzeichen der Pharmacia, Uppsala, Schweden).

Vorzugsweise wird anschliessend der beladene Ionenaustauscher ein oder mehrere Male mit Wasser gewaschen.

Zur Elution des Nikkomycin-Komponentenpaares (X) + (Z) vom basischen Ionenaustauscher eignen sich verdünnte Lösungen von niedrigen aliphatischen Carbonsäuren, z.B. Essigsäure, die auf einfache Weise, z.B. durch Abdampfen oder Abschleppen mit anderen geeigneten Lösungsmitteln leicht zu entfernen sind, ohne dass dabei der pH-Wert in Richtung eines sauren Milieus so stark verändert wird, dass Hydrolyse, insbesondere des Komponentenpaares (X) + (Z), eintritt. Die Konzentration liegt vorzugsweise im Bereich von 0,1 - 10%, insbesondere bei 1-5% (Gew.-%).

Die Elution des Nikkomycin (X) und (Z)-Gemisches kann nach den allgemein üblichen Methoden, z.B. durch einfaches Ausrühren mit Lösungen steigender Säurekonzentration oder durch Gradientenelution über eine Säule erfolgen.

Beim Ausrühren des mit dem Nikkomycin-Gemisch (X)+(Z) beladenen basischen Ionenaustauschers mit Lösungen steigender Säurekonzentration erhält man in den ersten Fraktionen eine bevorzugte Anreicherung von Nikkomycin (Z), während später bei höheren Säurekonzentrationen hauptsächlich Nikkomycin (X) angereichert wird.

Das Mischungsverhältnis von Nikkomycin (X) und (Z) kann also je nach Wunsch durch Abtrennen der jeweils zutreffenden Fraktionen stark beeinflusst werden.

Die Isolierung des Gemisches der Nikkomycine (X) und (Z) aus dem Eluat erfolgt nach den in der Biochemie allgemein üblichen Methoden, z.B. durch Verdampfen des Lösungsmittels, vorzugsweise unter vermindertem Druck oder durch Gefriertrocknung.

Wie bereits oben dargelegt wurde, kann man bei der Nikkomycin-Aufarbeitung auch direkt von einem Kulturfiltrat der mikrobiellen Erzeugung ausgehen oder auch eine angereicherte Rohnikkomycin-Lösung einsetzen, wobei zur Anreicherung in einer vorgeschalteten Stufe ein Kulturfiltrat mit einem sauren Ionenaustauscher behandelt wird, bei nachfolgender Elution mit einer schwachen Base. Dieser Schritt soll im folgenden näher erläutert werden.

Das bei der mikrobiellen Erzeugung des Nikkomycins in bekannter Weise erhaltene Kulturfiltrat wird auf einen pH-Wert von 2 bis 5, vorzugsweise 3,5 bis 4,5, insbesondere 4 durch Säurezugabe eingestellt. Als Säuren sind hierbei solche Säuren geeignet, welche die obigen pH-Werte einzustellen vermögen. Vorzugsweise werden niedere aliphatische Carbonsäuren, insbesondere Essigsäure verwendet.

Diese Lösung wird nach allgemein üblichen Methoden mit einem sauren Ionenaustauscher behandelt.

Man kann die Nikkomycine (X) und (Z) z.B. durch einfaches Ausrühren mit dem Ionenaustauscher oder durch Auftragen oder Durchfluss der Nikomycin-Lösung durch eine mit Ionenaustauscher gefüllte Säule binden.

Als saure Ionenaustauscher eignen sich vorzugsweise die üblichen makroporösen oder gelförmigen Ionenaustauscher aus mit Divinylbenzol vernetzten Polystyrolharzen mit Sulfonsäuregruppierungen z.B. Lewatit SC 104 (Warenzeichen der BAYER AG, Leverkusen, Deutschland, BRD) und Dowex 50 WX 4 (Warenzeichen der Dow Chemicals USA).

Vorzugsweise wird der beladene Ionenaustauscher ein oder mehrere Male mit Wasser gewaschen.

Zur Elution des Nikkomycin-Komponentenpaares (X) und (Z) von sauren Ionenaustauschern eignen sich schwache Basen, beispielsweise verdünntes Ammoniak. Die Konzentration liegt hierbei vorzugsweise im Bereich von 0,01 – 0,1 N, insbesondere bei 0,04 – 0,06 N.

Das Eluat oder eine Lösung des aus dem Eluat isolierten Rohnikkomycins wird, wie oben beschrieben, auf den geforderten pH-Wert gebracht und mit dem basischen Ionenaustauscher behandelt.

Alle auf diesen Wegen erhaltenen Nikkomycine sind als Ausgangsstoffe für das erfindungsgemässe Verfahren in hervorragender Weise geeignet.

Überraschenderweise zeigen die erfindungsgemässen neuen Nikkomycin-Schwermetallsalze eine gegenüber den «freien» Nikkomycinen überlegene Langzeitwirkung insbesondere bei einer feuchten Umgebung, z.B. bei einer regenreichen Witterung. Die neuen Verbindungen stellen somit eine wertvolle Bereicherung auf dem Gebiet der Schädlingsbekämpfungsmittel dar.

Ein weiterer Gegenstand der Erfindung ist die Verwendung der neuen Wirkstoffe zur Bekämpfung von Schädlingen, vorzugsweise von schädlichen Pilzen und Arthropoden, wie Insekten und Spinnentieren. Besonders bevorzugt können die neuen Wirkstoffe als Akarizide eingesetzt werden.

Schädlingsbekämpfungsmittel, welche die neuen Wirkstoffe enthalten, deren Herstellung und Verwendung, sind ebenfalls Teile der Erfindung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-Äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirkstoffe eignen sich bei guter Pflanzenverträglichkeit und günstiger Warmblütertoxizität zur Bekämpfung von Pilzen und Arthropoden, wie Insekten und Spinnentieren insbesondere von Spinnmilben, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören beispielsweise:

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chroioptes spp., Sacroptes spp., Tarsonemus spp., Bryobia praetoiosa, Panonychus spp. und Tetranychus spp.

Die erfindungsgemässen Wirkstoffe zeigen auch eine gute fungizide Wirkung, insbesondere gegen Rosterkrankungen an verschiedenen Kulturpflanzen wie Puccinia-Arten, Uromyces-Arten, Phragmidium mucronatum, Botrytis-Arten, vor allem Botrytis cinerea bei Wein, Erdbeeren und Gemüsekulturen, gegen Sclerotinia-Arten, gegen echte Mehltaupilze, wie Erysiphe, und Sphaerotheca-Arten. Ferner können die erfindungsgemässen Wirkstoffe auch gegen Venturia-Arten, Alternaria-Arten, Pellicularia sasakii, Pyricularia oryzae und Cercospora-Arten eingesetzt werden. Ausser der Behandlung der oberirdischen Pflanzenteile können mit den erfindungsgemässen Wirkstoffen auch Krankheitserreger bekämpft werden, die die Pflanzen vom Boden her angreifen oder mit dem Saatgut übertragen werden.

Geeignete Formulierungen der neuen Nikkomycin-Salze sollen anhand der folgenden Beispiele erläutert werden. Die Formulierungen werden durch gründliches Vermischen der aufgeführten Bestandteile erhalten. Alle %-Angaben beziehen sich auf Gewichtsprozente.

Formulierungsbeispiel 1:
25,0 % Nikkomycin-Zn-Salz (gemäss Beispiel 1)
1,5 % Na-Alkylarylsulfonat
8,0 % Na-Alkylarylsulfonat, kondensiert
35,0 % Adipinsäure
10,0 % Zinksulfat
Rest % Quarzmehl

Formulierungsbeispiel 2:
25,0 % Nikkomycin-Cu-Salz (gemäss Beispiel 3)
10,0 % Myristylalkohol C 14
10,0 % Zinksulfat
15,0 % Adipinsäure
8,0 % Na-Alkylarylsulfonat, kondensiert
10,0 % synth. Kieselsäure
Rest % Quarzmehl

Formulierungsbeispiel 3:
12,5 % Nikkomycin-Zn-Salz (gemäss Beispiel 2)
12,5 % Nikkomycin-Cu-Salz (gemäss Beispiel 3)
1,0 % Na-Alkylarylsulfonat
5,0 % Na-Alkylarylsulfonat, kondensiert
10,0 % synth. Kieselsäure
Rest % Quarzmehl

Formulierungsbeispiel 4:
12,5 % Nikkomycin-Cu-Salz (gemäss Beispiel 3)
1,0 % Na-Alkylarylsulfonat
5,0 % Na-Alkylarylsulfonat, kondensiert
10,00 % synth. Kieselsäure
Rest % Quarzmehl

Die überraschende Überlegenheit der neuen Wirkstoffe gegenüber dem vorbekannten «freien» Nikkomycin in feuchtem bis nassem Milieu kann durch das folgende Vergleichsbeispiel gezeigt werden:

1. Methodenbeschreibung
Man lässt in einem von Raupenleim umgrenzten Feld auf einem Blatt einer Bohnenpflanze von weiblichen Tetranychus urticae Eier ablegen und entfernt die Weibchen wieder. Die so vorbereiteten Blätter taucht man in die entsprechend konzentrierten wässrigen Suspensionen der als WP formulierten Nikkomycin-Salze, bzw. in die entsprechend konzentrierten Nikkomycin-Lösungen. Einen Tag nach Behandlung wird mit einer 12,5 mm entsprechenden Regenmenge mit destilliertem Wasser beregnet.

Nach Beendigung der Versuchszeit werden in jedem Versuchsglied die überlebenden Spinnmilben ausgezählt und zu der Ausgangspopulation in Beziehung gesetzt. Man erhält die Abtötung in %.

2. Versuchsergebnisse

| Wirkstoff | Wirkstoff Gew.-% (bezogen auf Nikkomycin) | % Abtötung nach 12 Tagen |
|---|---|---|
| Versuch a) | | |
| Nikkomycin-Zn-Salz, WP (Formulierbeispiel 1) | 0,02 | 100 |
| Nikkomycin | 0,02 | 16 |

| Wirkstoff | Wirkstoff Gew.-% (bezogen auf Nikkomycin) | % Abtötung nach 12 Tagen |
|---|---|---|
| Versuch b) Nikkomycin-Cu-Salz; WP (Formulierungsbeispiel 4) | 0,02 | 100 |
| Nikkomycin | 0,02 | 44 |

Die Herstellung der erfindungsgemässen Nikkomycin-Salze sei anhand der folgenden Beispiele erläutert, wobei alle %-Angaben Gewichtsprozente bedeuten:

Beispiel 1

500 g Nikkomycingemisch (55% [X] + [Z] Gehalt) werden in 2 Liter Wasser gelöst und die erhaltene wässrige Lösung mit Natronlauge auf pH 6,5 bis 7,0 eingestellt. Anschliessend werden langsam 250 ml 2n $ZnSO_4$-Lösung hinzugegeben, wobei der pH-Wert der Lösung mit Natronlauge zwischen pH 6,5 bis 7,0 gehalten wird.

Das Reaktionsgemisch wird gekühlt und der Niederschlag abgesaugt. Anschliessend wäscht man den Niederschlag mit Wasser, Wasser/Aceton und schliesslich mit Aceton.

Eine Nachfällung mit 100 ml 2n $ZnSO_4$-Lösung aus der Mutterlauge ergibt nochmals einen geringen Niederschlag, der analog behandelt wird.

Man erhält 310 g Nikkomycin-Zn-Salz mit einem Nikkomycin (X) und (Z)-Gehalt von 85,6% (HPLC) und mit einem Zinkgehalt von 8,3%. Die Ausbeute beträgt 96,5% bezogen auf eingesetztes Nikkomycin. Zersetzungspunkt: 255°C.

Beispiel 2

10 Liter Nikkomycin-Eluat (enthaltend 62,6 g Nikkomycin) aus der Aufarbeitungsstufe mit dem basischen Ionenaustauscher gemäss Ausgangsmaterial-Vorschrift 2 werden mit 200 ml 1N $ZnSO_4$-Lösung versetzt und der pH-Wert der Lösung mit Natronlauge zwischen pH 6,5 bis 7,0 gehalten.

Die Aufarbeitung erfolgt wie in Beispiel 1. Man erhält ein 80–90%iges Nikkomycin-Zn-Salz mit einer Ausbeute von 70–90%. Man erhält 72 g Nikkomycin-Zn-Salz.

Beispiel 3

10 g Nikkomycingemisch (50% [X]-und [Z]-Gehalt nach HPLC) wird in 50 ml $H_2O$ gelöst und die erhaltene wässrige Lösung mit Natronlauge auf pH 6,5 bis 7,0 eingestellt.

Dazu werden langsam 14 ml 1 N $CuSO_4$-Lösung hinzugegeben, wobei der pH-Wert der Lösung mit NaOH-Lösung zwischen pH 6,5 bis 7,0 gehalten wird. Es wird gekühlt und abgesaugt. Danach wäscht man den Niederschlag mit Wasser, Wasser/Aceton und schliesslich mit Aceton. Man erhält 7,3 g Nikkomycin-Cu-Salz mit einem Nikkomycingehalt an (X) und (Z) von 62% (nach HPLC). Die Ausbeute beträgt 90% bezogen auf eingesetzten Nikkomycin (X) und (Z).

Die Herstellung eines für das erfindungsgemässe Verfahren geeigneten Ausgangsmaterials sei im folgenden beispielhaft erläutert.

Ausgangsmaterial-Vorschrift 1:
Stufenweise Elution im Batch-Verfahren

1 kg Rohnikkomycin / ca. 15% (X) + (Z) / wird in 4 Ltr. entmineralisiertem $H_2O$ gelöst und der pH-Wert der Lösung durch Zugabe von Essigsäure auf pH 6,5–7,0 eingestellt. Diese Lösung wird zu 10 kg des in 16 Ltr. entmineralisiertem Wasser suspendierten schwach basischen Ionenaustauschers (gewaschen und mit Essigsäure auf pH 7,0 eingestellt) gegeben, wobei man den pH-Wert der Lösung durch Zugabe von Essigsäure bei 6,5–7,0 hält und 1 Stunde rührt. Danach wird abgesaugt und das beladene Austauscherharz dreimal je 15 Minuten mit 10 Ltr. Wasser ausgerührt. Das gewaschene Ionenaustauscherharz wird daraufhin stufenweise jeweils ½ Stunde mit je 10 Ltr. Essigsäure steigender Konzentration (1%, 2%, 3%, 4%, 5%, 10%) ausgerührt, wobei der pH-Wert durch Zugabe von Essigsäure während der jeweiligen Ausrührungsstufe konstant gehalten wird. Man erhält ein bis zu 70%iges Nikkomycin (X) + (Z) mit 80%iger Ausbeute.

Ausgangsmaterial-Vorschrift 2:
Elution mit Gradient

5 kg Rohnikkomycin (15–30%) werden in 20 Ltr. entmineralisiertem Wasser gelöst und der pH-Wert mit Essigsäure auf 6,5–7,0 eingestellt. In einer Vorlage mit Rührer werden 80 Ltr. entmineralisiertes Wasser und 50 kg des schwach basischen Ionenaustauschers (gewaschen und mit Essigsäure auf pH 7,0 eingestellt) vorgelegt. Die Rohnikkomycin-Lösung wird dazugegeben und die Mischung 60 Minuten lang gerührt. Der pH-Wert wird durch Zugabe der entsprechenden Menge an Essigsäure auf einen pH-Wert von 6,5–7,0 gehalten. Der beladene Ionenaustauscher wird zweimal mit je 100 Ltr. entmineralisiertem Wasser gewaschen. Danach wird der Ionenaustauscher in eine konische Glassäule eingefüllt. Die Säule wird eluiert mit einem linearen Gradienten, hergestellt aus 600 Ltr. entmineralisiertem Wasser und 600 Ltr. 10%iger Essigsäure. Der Säulenvorlauf wird verworfen. Die Elution des Nikkomycins wird anhand des pH-Wertes und der Leitfähigkeit verfolgt.

Der Reinheitsgrad beträgt ca. 90% mit einer Ausbeute von 70–80%.

Der Nikkomycin-Gehalt wird jeweils mit der Hochdruck-Flüssigchromatographie (HPLC) nach üblichen Methoden bestimmt.

**Patentansprüche**

1. Cr-, Fe-, Co-, Ni-, Cu- und Zn-Salze von Nikkomycin.
2. Cu- und Zn-Salze von Nikkomycin.
3. Schwermetallsalze gemäss den Ansprüchen 1 und 2 von Nikkomycin (X) und Nikkomycin (Z).

4. Verfahren zur Herstellung von Cr-, Fe-, Co-, Ni-, Cu- und Zn-Salzen von Nikkomycin, dadurch gekennzeichnet, dass man Nikkomycin mit einem in Wasser löslichen Cr-, Fe-, Co-, Ni-, Cu- oder Zn-Salz in wässrigem Milieu umsetzt.

5. Cr-, Fe-, Co-, Ni-, Cu- und Zn-Salze von Nikkomycin, erhältlich durch die Umsetzung von Nikkomycin mit in Wasser löslichen Cr-, Fe-, Co-, Ni-, Cu- oder Zn-Salzen.

6. Cr-, Fe-, Co-, Ni-, Cu- und Zn-Salze von Nikkomycin, erhältlich durch die Umsetzung von Nikkomycin, vorzugsweise von Nikkomycin (X) und (Z), mit in Wasser löslichen Cr-, Fe-, Co-, Ni-, Cu- und Zn-Salzen, vorzugsweise deren Chloride, Sulfate oder Acetate, in wässrigem Medium.

7. Cu- und Zn-Salze von Nikkomycin, vorzugsweise von Nikkomycin (X) und (Z), erhältlich durch die Umsetzung von Nikkomycin, vorzugsweise Nikkomycin (X) und (Z), mit $CuSO_4$ und $ZnSO_4$ in wässrigem Medium.

8. Verwendung der Schwermetallsalze von Nikkomycin gemäss den Ansprüchen 1 bis 3 und 5 bis 7 zur Schädlingsbekämpfung.

9. Schädlingsbekämpfungsmittel, enthaltend wenigstens ein Schwermetallsalz von Nikkomycin gemäss den Ansprüchen 1 bis 3 und 5 bis 7.

## Claims

1. Cr salts, Fe salts, Co salts, Ni salts, Cu salts and Zn salts of nikkomicin.

2. Cu salts and Zn salts of nikkomicin.

3. Heavy metal salts, according to Claims 1 and 2, of nikkomicin (X) and nikkomicin (Z).

4. Process for the preparation of Cr salts, Fe salts, Co salts, Ni salts, Cu salts and Zn salts of nikkomicin, characterised in that nikkomicin is reacted with a water-soluble Cr salt, Fe salt, Co salt, Ni salt, Cu salt or Zn salt in an aqueous medium.

5. Cr salts, Fe salts, Co salts, Ni salts, Cu salts and Zn salts of nikkomicin, obtainable by reacting nikkomicin with water-soluble Cr salts, Fe salts, Co salts, Ni salts, Cu salts or Zn salts.

6. Cr salts, Fe salts, Co salts, Ni salts, Cu salts and Zn salts of nikkomicin, obtainable by reacting nikkomicin, preferably nikkomicin (X) and nikkomicin (Z), with water-soluble Cr salts, Fe salts, Co salts, Ni salts, Cu salts and Zn salts, preferably the chlorides, sulphates or acetates, in an aqueous medium.

7. Cu salts and Zn salts of nikkomicin, preferably of nikkomicin (X) and nikkomicin (Z), which are obtainable by reacting nikkomicin, preferably nikkomicin (X) and nikkomicin (Z), with $CuSO_4$ or $ZnSO_4$ in an aqueous medium.

8. Use of the heavy metal salts of nikkomicin according to Claims 1 to 3 and 5 to 7 for combating pests.

9. Pest-combating agents containing at least one heavy metal salt of nikkomicin according to Claims 1 to 3 and 5 to 7.

## Revendications

1. Sels de Cr, Fe, Co, Ni, Cu et Zn de nikkomycine.

2. Sels de Cu et Zn de nikkomycine.

3. Sels de métaux lourds selon les revendications 1 et 2 de la nikkomycine X et de la nikkomycine Z.

4. Procédé de préparation des sels de Cr, Fe, Co, Ni, Cu et Zn de nikkomycine, caractérisé en ce que l'on fait réagir la nikkomycine avec un sel hydrosoluble de Cr, Fe, Co, Ni, Cu ou Zn en milieu aqueux.

5. Sels de Cr, Fe, Co, Ni, Cu et Zn de nikkomycine susceptibles d'être obtenus par réaction de la nikkomycine avec des sels hydrosolubles de Cr, Fe, Co, Ni, Cu ou Zn.

6. Sels de Cr, Fe, Co, Ni, Cu et Zn de nikkomycine susceptibles d'être obtenus en faisant réagir de la nikkomycine, de préférence de la nikkomycine X et Z avec des sels hydrosolubles de Cr, Fe, Co, Ni, Cu et Zn, de préférence leurs chlorures, sulfates ou acétates, en milieu aqueux.

7. Sels de Cu et Zn de nikkomycine, de préférence de nikkomycine X et Z, susceptibles d'être obtenus par réaction de la nikkomycine, de préférence la nikkomycine X et Z, avec $CuSO_4$ et $ZnSO_4$ en milieux aqueux.

8. Utilisation des sels de métaux lourds de nikkomycine selon les revendications 1 à 3 et 5 à 7 dans la lutte contre les parasites.

9. Produits pesticides contenant au moins un sel de métal lourd de la nikkomycine selon les revendications 1 à 3 et 5 à 7.